# EUROPÄISCHE PATENTANMELDUNG

(11) **EP 0 839 553 A2**
(43) Veröffentlichungstag der Anmeldung: **06.05.1998**
(21) Anmeldenummer: 97118119.3
(22) Anmeldetag: 18.10.1997
(51) Int. Cl.: A61N 1/05

(54) **Elektrode für die Implantation in Körpergewebe, Verfahren zu ihrer Herstellung und ihre Verwendung (I)**

(30) Priorität: 02.11.1996 DE 19645155
(71) Anmelder: W.C. Heraeus GmbH, D-63450 Hanau (DE)
(72) Erfinder: Herklotz, Günther, Dr., 63486 Bruchköbel (DE); Schölz, Friedhold, 63517 Rodenbach (DE); Giesel, Thomas, 63526 Erlensee (DE)
(74) Vertreter: Kühn, Hans-Christian

(57) **Zusammenfassung**

An einer Elektrode, deren aktiver Bereich eine aus mehreren Platin-Schichten bestehende poröse, strukturierte Beschichtung mit großer Oberfläche aufweist, bildet sich eine hohe Doppelschichtkapazität aus. Die Elektrode ist besonders zur Verwendung in Herzschrittmachern geeignet.

## Beschreibung

Die Erfindung betrifft eine Elektrode für die Implantation in Körpergewebe aus einem metallischen Grundkörper und einer den aktiven Bereich der Elektrode bildenden porösen Beschichtung, ein Verfahren zu ihrer Herstellung und ihre Verwendung.

Die Erfindung betrifft besonders eine Elektrode für Herzschrittmacher.

Implantierbare Elektroden dieser Art bestehen in den meisten Fällen aus einem Elektrodenschaft mit einer isolierten Kabelzuleitung und aus einem Elektrodenkopf - dem aktiven oder wirksamen Bereich der Elektrode. Das Elektrodenmaterial muß körperverträglich sein, um die Bildung von Bindegewebsschichten soweit wie möglich zu verhindern und die Reizschwelle möglichst konstant zu halten. An der Phasengrenze Elektrode/Körperflüssigkeit soll sich eine hohe Doppelschichtkapazität ausbilden; der Polarisationsanstieg während der Reiz- oder Stimulationsimpulse soll kleiner als 0,1 V sein. Die geforderte hohe Doppelschichtkapazität wirkt sich bei Reizelektroden (Stimulationselektroden) günstig aus, weil durch den aufgeprägten Strom nur geringe Potentialänderungen entstehen, elektrochemische Reaktionen mit der Hörperflüssigkeit weitgehend unterbleiben und der Energieaufwand gering ist. Im Falle von Sensoren, bei denen nur ein kleiner Meßstrom fließt, erleichtert eine hohe Kapazität der Elektroden die Erfüllung der Anforderungen, die an die Eingangsimpedanz von Verstärkern zu stellen sind; außerdem wird das Rauschen reduziert (DE 33 00 668 A1).

Implantierbare Elektroden mit porösen Oberflächen sind zum Beispiel aus GB 1 552 388, EP 0 043 461 A1, EP 0 054 781 B1 und DE 42 07 368 A1 bekannt.

In GB 1 552 388 werden kardiovaskuläre Prothesen und Implantate, beispielsweise Teile für Herzschrittmacherelektroden, mit porösen metallischen Überzügen beschrieben. Die Überzüge bestehen aus miteinander verbundenen metallischen Teilchen, die ein sich gleichmäßig verteilendes Porennetz bilden. Die Überzüge werden durch Aufbringen der metallischen Teilchen auf die aufgerauhte Oberfläche der metallischen Grundkörper und anschließendes Sintern hergestellt. Die Art und Weise des Aufbringens der metallischen Teilchen hängt von deren Korngröße ab und kann zum Beispiel durch Aufsprühen einer viskosen Mischung aus metallischen Teilchen und Bindemittel und nachfolgendes Trocknen erfolgen. Die für das Sintern günstige Temperatur hängt ebenfalls von der Korngröße der metallischen Teilchen ab und liegt zwischen 1100 und 1200° C.

Aus EP 0 043 461 A1 ist eine Elektrode für Herzstimulatoren bekannt, deren Kopf aus einer mit einem Überzug aus Platin-Schwamm versehenen Platin-Iridium-Legierung besteht. Die Dicke des Überzugs beträgt etwa 0,1 Millimeter, der Porendurchmesser etwa 40 Mikrometer. Der Überzug aus Platin-Schwamm wird durch galvanisches Abscheiden erzeugt und kann durch Sintern bei etwa 1300° C verdichtet werden.

In EP 0 054 781 B1 wird eine poröse Elektrode zur Implantation in organisches Gewebe vorgeschlagen, die als Grundkörper einen aus elektrisch leitenden Teilchen hergestellten Sinterkörper aufweist. Eine vorteilhafte Ausführungsform der Elektrode besteht aus einem Tantal-Sinterkörper, dessen Oberfläche teilweise mit einer dünnen Tantaloxid-Schicht und teilweise mit einer aus einem handelsüblichen Platin-Bad galvanisch abgeschiedenen Platin-Schicht versehen ist. Der größte Teil der Poren ist zum Körpergewebe hin offen; die Porengröße hat vorteilhafterweise einen Durchmesser im Bereich von 10 - 50 Mikrometer. Die Elektrode besitzt eine niedrige Reizschwelle und stellt besonders eine Herzschrittmacherelektrode dar.

Aus DE 42 07 368 A1 ist eine zur Anwendung als Herzschrittmacher- oder Neurostimulationselektrode geeignete Stimulationselektrode aus einem Grundkörper und einer porösen Oberflächenbeschichtung aus inertem Material mit nur sehr geringer Oxidationsneigung bekannt. Das inerte Material kann ein inertes Element, eine inerte chemische Verbindung und/oder eine inerte Legierung sein und besteht besonders aus einem Carbid, Nitrid oder Carbonitrid, aus Gold, Silber, Iridium, Platin, einer Legierung dieser Metalle oder aus Kohlenstoff und wird durch Vakuumbeschichtungsverfahren (reaktive Kathodenzerstäubung oder Ionenplattierung) auf den Grundkörper aufgebracht. Wie die elektronenmikroskopisch vergrößerte Darstellung zeigt, besitzt die Stimulationselektrode ein blumenkohlartiges Äußeres. Durch die sich nach außen hin verfeinernde Struktur wird eine mikroskopische Oberfläche erzielt, die um ein Vielfaches größer ist als der makroskopische Flächenbereich. Da die aktive Oberfläche der Elektrode sehr groß ist, kann die zur Stimulation erforderliche Energiemenge gering gehalten werden, so daß die Betriebszeit (Lebensdauer) der die Elektrode enthaltenden Implantate verlängert wird. Mit den Elektroden - sie können monopolar, bipolar oder multipolar sein - kann auch eine infrakardiale Impedanzmessung zur Erfassung der Herzaktivität erfolgen. Eine mit Iridiumnitrid beschichtete Elektrode besitzt in dem für den Empfang von aus dem Herzen aufnehmenden Signalen besonders wichtigen niederfrequenten Bereich eine sehr niedrige Phasengrenzimpedanz.

Der Erfindung liegt die Aufgabe zugrunde, eine Elektrode für die Implantation in Körpergewebe zur Verfügung zu stellen, die aus einem metallischen Grundkörper und einer den aktiven Bereich der Elektrode bildenden porösen Beschichtung besteht. Die poröse Beschichtung soll so strukturiert sein, daß eine große aktive Oberfläche und eine sich an der Elektrode ausbildende hohe Doppelschichtkapazität erreicht werden. Außerdem soll ein Verfahren zur Herstellung der Elektrode, die besonders zur Verwendung in Herzschrittmachern geeignet sein soll, angegeben werden.

Die die Lösung der Aufgabe darstellende Elektrode ist erfindungsgemäß dadurch gekennzeichnet, daß die Beschichtung aus einer direkt auf dem Grundkörper befindlichen porösen, stengelartig strukturierten Platin-Schicht und einer darauf befindlichen porösen Schicht aus feindispersem Platin besteht.

Besonders bewährt hat sich die Elektrode, wenn die Dicke der porösen Beschichtung 10 - 200 Mikrometer, vorzugsweise 50 - 100 Mikrometer, beträgt und die Dicke der porösen, stengelartig strukturierten Platin-Schicht 80 - 90 % der Dicke der gesamten Beschichtung ausmacht.

Die Charakterisierung der die Beschichtung bildenden Schichten im Sinne der Erfindung als poröse, stengelartig strukturierte Platin-Schicht und als poröse Schicht aus feindispersem Platin beruht auf der mikroskopischen Betrachtung der Beschichtung.

Der metallische Grundkörper besteht aus einem körperverträglichen Übergangsmetall oder einer körperverträglichen Übergangsmetall-Legierung. Als besonders geeignet haben sich Titan und Platin-Iridium-Legierungen mit bis zu 20 Gewichts-% Iridium, zum Beispiel eine Legierung aus 90 Gewichts-% Platin und 10 Gewichts-% Iridium, erwiesen.

Weiterhin wird die Aufgabe der Erfindung durch ein Verfahren gelöst, nach dem die beanspruchte Elektrode hergestellt werden kann.

Dieses Verfahren ist erfindungsgemäß dadurch gekennzeichnet, daß auf das den aktiven Elektroden-Bereich bildende Teil des metallischen Grundkörpers eine Platin-Pulver enthaltende pastenförmige Zubereitung und darauf Platin-Pulver aufgebracht, der so behandelte Grundkörper getrocknet und gesintert und anschließend eine poröse Schicht aus feindispersem Platin aufgebracht wird.

Das erfindungsgemäße Verfahren wird vorzugsweise so ausgeführt, daß die Dicke der fertigen Beschichtung 10 - 200 Mikrometer, besonders 50-100 Mikrometer, beträgt. Dabei soll das Auftragen und Sintern von pastenförmiger Zubereitung und Platin-Pulver so erfolgen, daß die Dicke der auf diese Weise erzeugten porösen, stengelartig strukturierten Platin-Schicht 80 - 90 % der Dicke der gesamten Beschichtung ausmacht.

Das erfindungsgemäße Verfahren hat sich besonders bewährt, wenn das Aufbringen von pastenförmiger Zubereitung und Platin-Pulver auf den metallischen Grundkörper und das Trocknen und Sintern mindestens einmal wiederholt werden und das Trocknen bei 100 - 200° C und das Sintern bei 400 - 1300° C erfolgt.

Für das Verfahren wird eine pastenförmige Zubereitung, die neben einem thermisch zersetzbaren organischen Bindemittel, besonders aus einem Cellulosederivat, und einem organischen Träger 20 - 80 Gewichts-% Platin-Pulver enthält, bevorzugt. Sowohl für die pastenförmige Zubereitung als auch zum Aufbringen darauf hat sich ein Platin-Pulver, dessen Teilchengröße 0,001 - 10 Mikrometer beträgt, als besonders geeignet erwiesen.

Die poröse Schicht aus feindispersem Platin wird vorzugsweise durch galvanisches Abscheiden aufgebracht und kann durch Sintern verdichtet werden (siehe EP 0 043 461 A1).

Der in dem Verfahren eingesetzte metallische Grundkörper besteht aus einem körperverträglichen Übergangsmetall oder einer körperverträglichen Übergangsmetall-Legierung, vorzugsweise aus Titan oder einer Platin-Iridium-Legierung mit bis zu 20 Gewichts-% Iridium, zum Beispiel einer Legierung aus 90 Gewichts-% Platin und 10 Gewichts-% Iridium.

Die Elektrode gemäß der Erfindung zeichnet sich durch die an der Grenzfläche Elektrode/Elektrolyt sich ausbildende hohe Doppelschichtkapazität aus, die im wesentlichen auf der den aktiven Bereich der Elektrode bildenden porösen Beschichtung mit der ihr eigenen Strukturierung beruht.

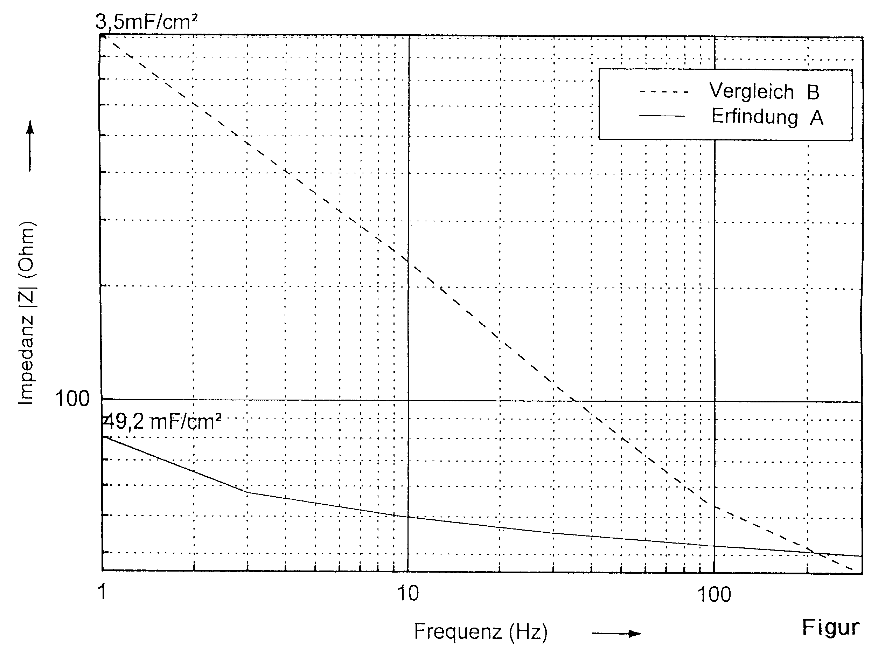

Für die Bestimmung der üblicherweise zur Charakterisierung implantierbarer Elektroden dienenden Doppelschichtkapazität bei einer Frequenz von 1 Hz wird als beispielhafte Ausführungsform der Erfindung eine Elektrode benutzt, deren Grundkörper aus Titan und deren aktiver Bereich aus einer porösen Beschichtung aus einer 50 Mikrometer dicken porösen, stengelartig strukturierten Platin-Schicht und einer darauf befindlichen 10 Mikrometer dicken porösen Schicht aus feindispersem Platin besteht. Zum Vergleich wird auch die Doppelschichtkapazität einer Elektrode aus Titan mit einer porösen Beschichtung aus feindispersem Platin ermittelt. Die zur Bestimmung der Doppelschichtkapazität angewandten Messungen der Impedanz [Ohm] in Abhängigkeit von der Frequenz [Hz] erfolgen in 0,15 n NaCl-Lösungen. In der Figur werden die in Abhängigkeit von der Frequenz gemessenen Impedanz-Werte dargestellt.

Die anhand der Impedanzmessungen für die erfindungsgemäße Elektrode (A) und für die Vergleichselektrode (B) bestimmten Doppelschichtkapazitäten werden in der Tabelle angegeben.

**Tabelle**

| **Elektrode** | **Doppelschichtkapazität bei 1 Hz [mF/cm**^{**2**}**]** |
|---|---|
| A Erfindung | 49,2 |
| B Vergleich | 3,5 |

## Patentansprüche

1. Elektrode für die Implantation in Körpergewebe aus einem metallischen Grundkörper und einer den aktiven Bereich der Elektrode bildenden porösen Beschichtung, dadurch gekennzeichnet, daß die Beschichtung aus einer direkt auf dem Grundkörper befindlichen porösen, stengelartig strukturierten Platin-Schicht und einer darauf befindlichen porösen Schicht aus feindispersem Platin besteht.

2. Elektrode nach Anspruch 1, dadurch gekennzeichnet, daß die stengelartig strukturierte Platin-Schicht gesintert ist.

3. Elektrode nach Anspruch 1 oder 2, dadurch gekennzeichnet, daß die Dicke der Beschichtung 10 - 200 Mikrometer beträgt.

4. Elektrode nach Anspruch 3, dadurch gekennzeichnet, daß die Dicke der Beschichtung 50 - 100 Mikrometer beträgt.

5. Elektrode nach einem der Ansprüche 1 bis 4, dadurch gekennzeichnet, daß die Dicke der stengelartig strukturierten Platin-Schicht 80 - 90 % der Dicke der Beschichtung ausmacht.

6. Elektrode nach einem der Ansprüche 1 bis 5, dadurch gekennzeichnet, daß der metallische Grundkörper aus einem körperverträglichen Übergangsmetall besteht.

7. Elektrode nach Anspruch 6, dadurch gekennzeichnet, daß der metallische Grundkörper aus Titan besteht.

8. Elektrode nach Anspruch 6, dadurch gekennzeichnet, daß der metallische Grundkörper aus einer Platin-Iridium-Legierung besteht.

9. Verfahren zur Herstellung der Elektrode nach einem der Ansprüche 1 bis 8, dadurch gekennzeichnet, daß auf das den aktiven Elektroden-Bereich bildende Teil des metallischen Grundkörpers eine Platin-Pulver enthaltende pastenförmige Zubereitung und darauf Platin-Pulver aufgebracht, der so behandelte Grundkörper getrocknet und gesintert und anschließend eine poröse Schicht aus feindispersem Platin aufgebracht wird.

10. Verfahren nach Anspruch 9, dadurch gekennzeichnet, daß das Aufbringen von pastenförmiger Zubereitung und Platin-Pulver und das Trocknen und Sintern mindestens einmal wiederholt werden.

11. Verfahren nach Anspruch 9 oder 10, dadurch gekennzeichnet, daß das Trocknen bei 100 - 200° C und das Sintern bei 400 - 1300° C erfolgt.

12. Verfahren nach einem der Ansprüche 9 bis 11, dadurch gekennzeichnet, daß die pastenförmige Zubereitung 20 - 80 Gewichts-% Platin-Pulver enthält.

13. Verfahren nach einem der Ansprüche 9 bis 12, dadurch gekennzeichnt, daß die Teilchengröße des Platin-Pulvers 0,001 - 10 Mikrometer beträgt.

14. Verfahren nach einem der Ansprüche 9 bis 13, dadurch gekennzeichnet, daß die pastenförmige Zubereitung ein organisches Bindemittel enthält.

15. Verfahren nach Anspruch 14, dadurch gekennzeichnet, daß das organische Bindemittel aus einem Cellulosederivat besteht.

16. Verfahren nach einem der Ansprüche 9 bis 15, dadurch gekennzeichnet, daß die poröse Schicht aus feindispersem Platin durch galvanisches Abscheiden aufgebracht wird,

17. Verfahren nach Anspruch 16, dadurch gekennzeichnet, daß die poröse Schicht aus feindispersem Platin gesintert wird.

18. Verfahren nach einem der Ansprüche 9 bis 17, dadurch gekennzeichnet, daß ein Grundkörper aus körperverträglichem Übergangsmetall eingesetzt wird.

19. Verfahren nach Anspruch 18, dadurch gekennzeichnet, daß ein Grundkörper aus Titan eingesetzt wird.

20. Verfahren nach Anspruch 18, dadurch gekennzeichnet, daß ein Grundkörper aus einer Platin-Iridium-Legierung eingesetzt wird.

21. Verwendung der Elektrode nach einem der Ansprüche 1 bis 8 in Herzschrittmachern.

22. Verwendung der Elektrode nach einem der Ansprüche 1 bis 8 als Stimulationselektrode.
